# EUROPEAN PATENT APPLICATION

(11) **EP 4 420 695 A1**
(43) Date of publication of application: **28.08.2024**
(21) Application number: 23158590.2
(22) Date of filing: 24.02.2023
(51) Int. Cl.: A61M 5/24, A61M 5/32, A61M 5/28, A61M 5/315

(54) **AUTO-INJECTOR WITH MOTOR BODY AND CASSETTE BODY**

(71) Applicant: Becton, Dickinson and Company, Franklin Lakes, NJ 07417-1880 (US)
(72) Inventor: BESSON, Nicolas, 38650 Treffort (FR)
(74) Representative: Germain Maureau

(57) **Abstract**

A drug delivery device includes an upper housing shell, a lower housing shell, and a syringe. The syringe includes a barrel, a stopper, and a needle, at least a portion of the syringe positioned within the lower housing shell. The drug delivery device further includes a drive assembly including a plunger body configured to move the stopper within the barrel upon actuation of the drive assembly, a cassette body configured to receive the syringe, and a motor body configured to engage the cassette body. The motor body includes a pair of sidewalls and a contact surface provided on each of the pair of sidewalls. The contact surface engages a corresponding contact surface of the cassette body.

## Description

### BACKGROUND OF THE INVENTION

### Field of the Invention

The present disclosure relates generally to a drug delivery device and, more specifically, to an auto-injector.

### Description of Related Art

Various types of automatic injection devices have been developed to allow drug solutions and other liquid therapeutic preparations to be administered by untrained personnel or to be self-injected. Generally, these devices include a reservoir that is pre-filled with the liquid therapeutic preparation, and some type of automatic needle-injection mechanism that can be triggered by the user. Many of these devices, such as auto-injectors, are designed so that the reservoir, such as a pre-filled syringe, is assembled into the device during assembly of the device. In addition to automatically deploying the needle-injection mechanism, many drug delivery devices also automatically shield the needle after use of the device to prevent any unintended contact with the needle.

Automatic injection devices include several interacting components that must slide and/or rotate relative to one another in order to effectively perform an injection. The relatively small tolerances necessary to achieve component fitment can be difficult to achieve with an injection molding process depending on the shape of the individual components. Poor fitment can lead to increased friction between components, sticking or jamming of components, and increased wear, all of which can adversely affect drug delivery. As such, there is an ongoing need in the art to optimize the fitment of components of drug delivery devices.

### SUMMARY OF THE INVENTION

Provided herein is a drug delivery device including an upper housing shell, a lower housing shell, and a syringe. The syringe includes a barrel, a stopper, and a needle, at least a portion of the syringe positioned within the lower housing shell. The drug delivery device further includes a drive assembly including a plunger body configured to move the stopper within the barrel upon actuation of the drive assembly, a cassette body configured to receive the syringe, and a motor body configured to engage the cassette body. The motor body includes a pair of sidewalls and a contact surface provided on each of the pair of sidewalls. The contact surface engages a corresponding contact surface of the cassette body.

In some embodiments, the contact surface of the motor body includes a planar, substantially vertical surface.

In some embodiments, the corresponding contact surface of the cassette body includes a planar, substantially vertical surface.

In some embodiments, the contact surface of the motor body continuously contacts the corresponding contact surface of the cassette body contact along a length of the contact surface of the motor body.

In some embodiments, the cassette body includes at least one motor body rib including the contact surface of the cassette body.

In some embodiments, the contact surface of the cassette body extends substantially the entire length of the at least one motor body rib.

In some embodiments, the at least contact surface of the motor body is provided on an inside face of the sidewall of the motor body.

In some embodiments, the motor body includes at least one protrusion configured to engage a corresponding wall surface of the cassette body.

In some embodiments, the cassette body includes a pair of sidewalls including the corresponding wall surface.

In some embodiments, the protrusion includes a curved outer surface.

In some embodiments, interaction of the at least one protrusion and the corresponding wall surface of the cassette body forces the contact surface of the motor body into engagement with the contact surface of the cassette body.

In some embodiments, contact between the at least one protrusion and the corresponding wall surface of the cassette body defines a predetermined distance between inside faces of the sidewalls of the motor body.

In some embodiments, contact between the at least one protrusion and the corresponding wall surface of the cassette body forces the sidewalls of the motor body parallel to one another.

In some embodiments, the at least one protrusion and the contact surface of the motor body is provided on opposite sides of the sidewall of the motor body.

In some embodiments, the at least one protrusion is provided on an outside face of the sidewall of the motor body.

These and other features and characteristics of drug delivery devices and methods of operation and functions of the related elements of structures and the combination of parts and economies of manufacture, will become more apparent upon consideration of the following description and the appended claims with reference to the accompanying drawings, all of which form a part of this specification. It is to be expressly understood, however, that the drawings are for the purpose of illustration and description only.

### BRIEF DESCRIPTION OF THE DRAWINGS

FIG. 1 is a perspective view of a drug delivery device according to non-limiting embodiments described herein;
FIG. 2 is a cross-sectional side view of the drug delivery device of FIG. 1;
FIG. 3 is a perspective view of a second subassembly of the drug delivery device of FIG. 1;
FIG. 4 is a perspective view of a first subassembly of the drug delivery device of FIG. 1;
FIG. 5 is a front cross-sectional view of the drug delivery device of FIG. 1 taken along line A-A of FIG. 2;
FIG. 6 is a perspective view of a motor body of the drug delivery device of FIG. 1;
FIG. 7 is a detail perspective view of the motor body of FIG. 5;
FIG. 8 is a front view of the motor body of FIG. 5;
FIG. 9 is a top perspective view of a cassette body of the drug delivery device of FIG. 1;
FIG. 10 is a bottom perspective view of the cassette body of FIG. 9;
FIG. 11 is a detail perspective view of the cassette body of FIG. 9; and
FIG. 12 is a cross-sectional detail view of the drug delivery device of FIG. 1.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of " 1 to 10" is intended to include all sub-ranges between (and including) the recited minimum value of 1 and the recited maximum value of 10, that is, having a minimum value equal to or greater than 1 and a maximum value of equal to or less than 10.

When used in relation to a component of a drug delivery device, the term "distal" refers to a portion of said component nearest to a patient. When used in relation to a component of a drug delivery device, the term "proximal" refers to a portion of said component farthest from the patient.

As used herein, the term "at least one of" is synonymous with "one or more of'. For example, the phrase "at least one of A, B, and C" means any one of A, B, and C, or any combination of any two or more of A, B, and C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C. Similarly, as used herein, the term "at least two of' is synonymous with "two or more of'. For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

International Patent Application Publication Nos. WO 2020/173991, WO 2020/173992, WO 2020/173993, WO 2020/173994, and WO 2020/173995 are incorporated herein by reference in their entirety.

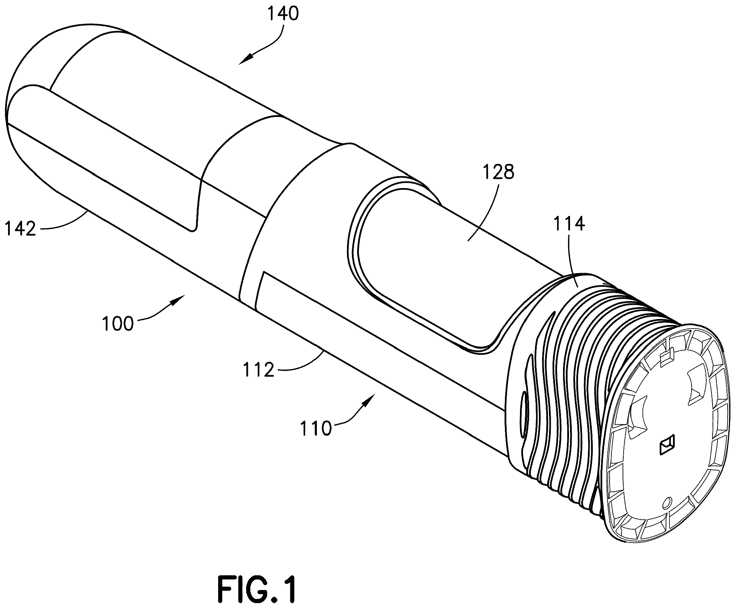

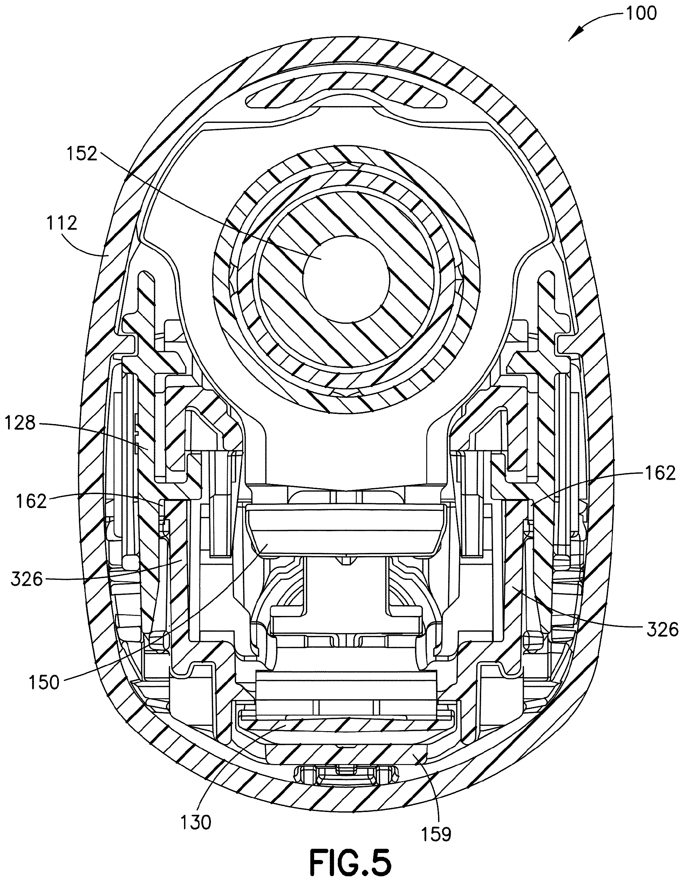

Turning to FIGS. 1-5, shown is a drug delivery device 100. The drug delivery device 100 includes a first subassembly 110, a second subassembly 140, and a syringe 116. The first subassembly 110 may include a lower housing shell 112, a cap 114, a syringe holder 118, a needle cover 124, and a cassette body 128. The second subassembly 140 may include a drive assembly including a plunger body 150. The plunger body 150 includes a plunger rod 152 and a spring guide member 154 including a drive member 156 and a drive opening 158. The second subassembly 140 may further include a motor body 159, a lever actuation member 130, and an upper housing shell 142. The syringe 116 may be received by the syringe holder 118 and includes a barrel 117, a stopper 122, a needle 120, and a rigid needle shield (RNS) 126. Although an RNS is utilized in the example shown in the accompanying drawings, other suitable needle shield arrangements may be utilized. The lower housing shell 112, the cassette body 128, and the upper housing shell 142 generally form a housing for receiving the various components of the drug delivery device 100, although other suitable housing arrangements may be utilized. The first subassembly 110 and the second subassembly 140 may be secured to each other during assembly by a locking clip, although other suitable arrangements may be utilized. The upper housing shell 142 includes an outer lip 143 configured to fit around an inner lip 113 of the lower housing shell 112 when the device 100 is assembled. The drug delivery device 100 may be an auto-injector, although the features described herein may be incorporated into other suitable drug delivery devices.

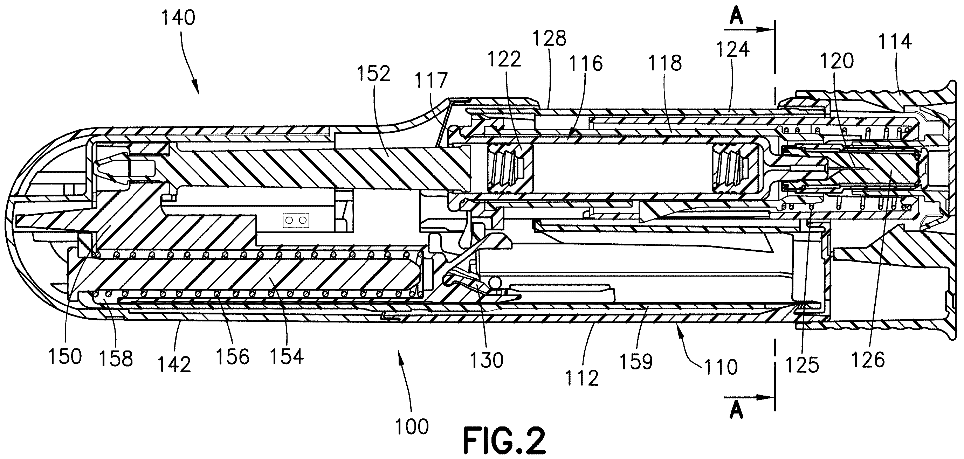

With continued reference to FIG. 2, the drive member 156 may be a compression spring received within a drive opening 158 defined by the plunger body 150, although other suitable drive members may be utilized, including, but not limited to, compressed gas, an electric motor, hydraulic pressure, other types of springs, etc. The drive member 156 engages the plunger body 150 and the motor body 159 and biases the plunger body 150 in a direction extending from the second subassembly 140 toward the first subassembly 110. The plunger body 150 defines a lever opening that receives the lever actuation member 130 and defines the rotation axis of the lever actuation member 130. The lever actuation member 130 prevents movement of the plunger body 150 when the lever actuation member 130 is in the locked position through engagement of the lever actuation member 130 with the motor body 159. Upon rotation of the lever actuation member 130 from the locked position to the released position, the lever actuation member 130 is disengaged from the motor body 159 thereby allowing the drive member 156 to move the plunger body 150 and the plunger rod 152 toward the first subassembly 110. The plunger rod 152 and the drive member 156 are spaced from and parallel to each other and extend in a longitudinal direction of the device 100.

The spring guide member 154 is secured to the upper housing shell 142 and received within the drive opening 158 of the plunger body 150. The drive member 156 is received by the spring guide member 154 such that the drive member 156 is positioned between the plunger body 150 and the spring guide member 154. In some embodiments, the drive assembly may also include a plunger rod cover (not shown) that receives the plunger rod 152 of the plunger body 150. The plunger rod cover may be configured to guide insertion of the plunger rod 152 into the barrel 117 of the syringe 116 and engage the stopper 122 to dispense the medicament from the barrel 117 of the syringe 116.

The drug delivery device 100 is configured to automatically deliver a dose of medicament from the syringe 116 to a patient upon actuation of the device 100. More specifically, upon actuation of the drug delivery device 100, the drive assembly is configured to engage the stopper 122 of the syringe 116, displace the syringe 116 such that the needle 120 pierces the skin of the patient, and displace the stopper 122 within the barrel 117 of the syringe 116 to deliver the medicament within the barrel 117. The drug delivery device 100 includes a storage position, a pre-use position, an actuation position, an injection position, and a post-use position, as described in International Patent Application Publication Nos. WO 2020/173991, WO 2020/173992, WO 2020/173993, WO 2020/173994, and WO 2020/173995.

The needle cover 124 is configured to shield the needle 120 of the syringe 116 from the patient when the device 100 is in the pre-use and the post-use positions. In particular, the needle cover 124 is moveable between a pre-use position, an actuation position, and a post-use positon, with a spring 125 biasing the needle cover 124 towards the pre-use position and the post-use position. The spring 125 may be positioned between the needle cover 124 and the syringe holder 118, although other suitable arrangements may be utilized.

The lever actuation member 130 is moveable between a locked position where movement of the drive assembly is prevented and a released position where movement of the drive assembly is allowed. More specifically, the lever actuation member 130 is rotatable about a rotation axis between the locked position and the released position. When the lever actuation member 130 is in the locked position, the lever actuation member 130 is engaged with the motor body 159 and the drive assembly to prevent movement of the drive assembly.

When the lever actuation member 130 is in the released position, which may be achieved by pressing the needle cover 124 onto the patient's skin at the site of injection, shifting the needle cover proximally into the lower housing shell 112, the lever actuation member 130 is disengaged from the motor body 159 thereby allowing movement of the drive assembly toward the syringe 116. The rotation axis of the lever actuation member 130 extends perpendicular to a longitudinal axis of the device 100, although other suitable arrangements may be utilized.

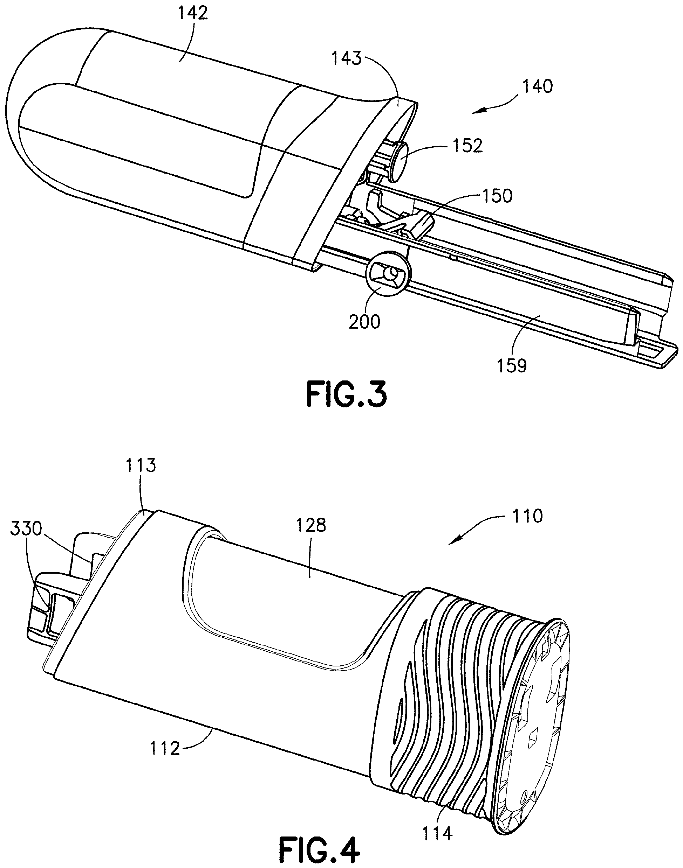

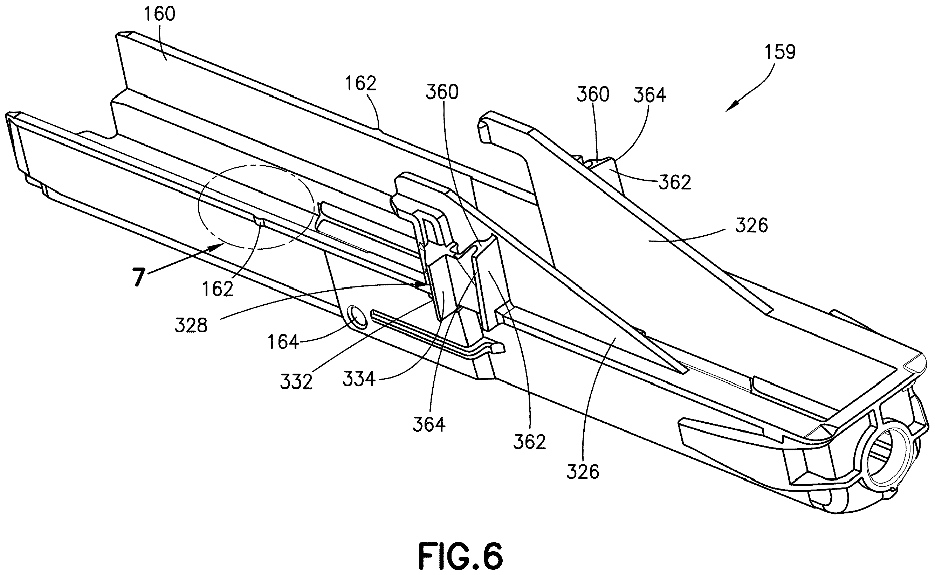

FIGS. 3 and 4 illustrate the second subassembly 140 and the first subassembly 110, respectively, as these subassemblies may be provided for final assembly of the device 100. As shown in FIG. 3, a safety pin 200 is inserted through apertures 164 (see FIG. 6) of the motor body 159. The apertures 164 are located in proximity to a distal end of the plunger body 150 so that with the safety pin 200 inserted through the apertures 164, the safety pin 200 prevents rotation of the lever actuation member 130 and unintentional actuation of the drive member 156. As will be described, the safety pin 200 is removed prior to or during final assembly of the device 100.

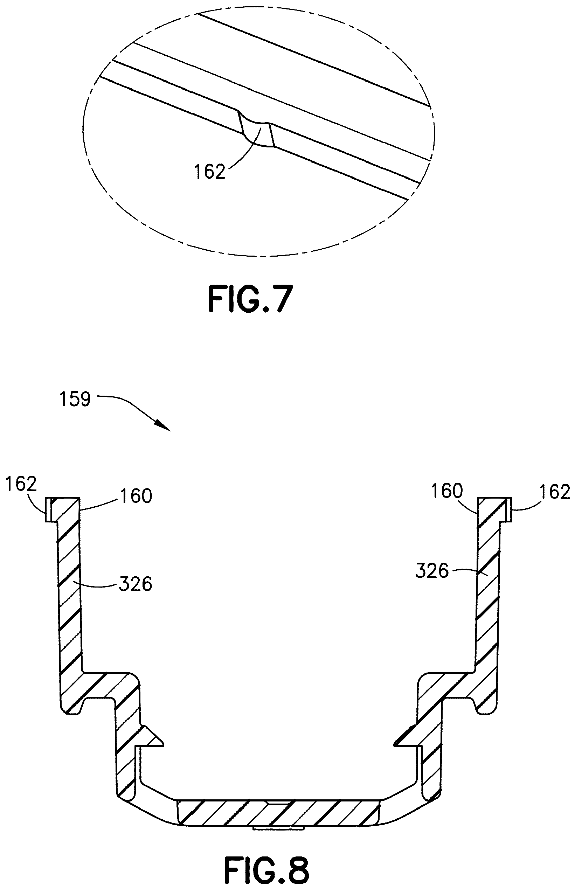

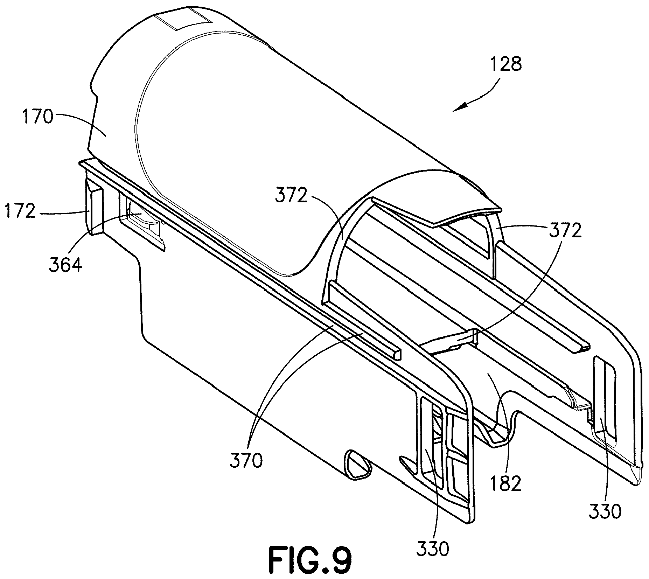

Referring now to FIGS. 6-8, the motor body 159 is generally U-shaped and includes a pair of opposing sidewalls 326 configured to receive the plunger body 150 therebetween. The motor body 159 defines the safety pin apertures 164 extending transversely through the sidewalls 326 to facilitate insertion of the safety pin 200 (see FIG. 3). The motor body 159 further includes at least one cassette clip 328 extending from the respective sidewalls 326 for engaging complementary openings 330 (see FIG. 9) defined by the cassette body 128. Engagement of the at least one cassette clip 328 with the openings 330 secures the motor body 159 to the cassette body 128, which is discussed in more detail below. The at least one cassette clip 328 includes an angled face 332 and a planar face 334, which are configured to allow insertion of the at least one cassette clip 328 into the corresponding openings 330 of the cassette body 128, but prevent easy removal of the cassette clips 328 once inserted into the openings 330 of the cassette body 128.

The motor body 159 is configured to be inserted in the upper housing shell 142 of the second subassembly 140. The motor body 159 includes at least one shell stop 360 extending outward from the opposing sidewalls 326 of the motor body 159. Each shell stop 360 has a planar proximal face 362 configured to engage an internal structure of the upper housing shell 142 to index the motor body 159 in the upper housing shell 142. The at least one shell stop 360 extends a sufficient distance outward from the sidewalls 326 of motor body 159 so that the at least one shell stop 360 remains at least partially engaged with the corresponding internal structure of the upper housing shell 142 when the sidewalls 326 of the motor body 159 are deflected inward during connection of the motor body 159 to the cassette body 128. In particular, each of the at least one shell stops 360 includes a cantilevered end 364.

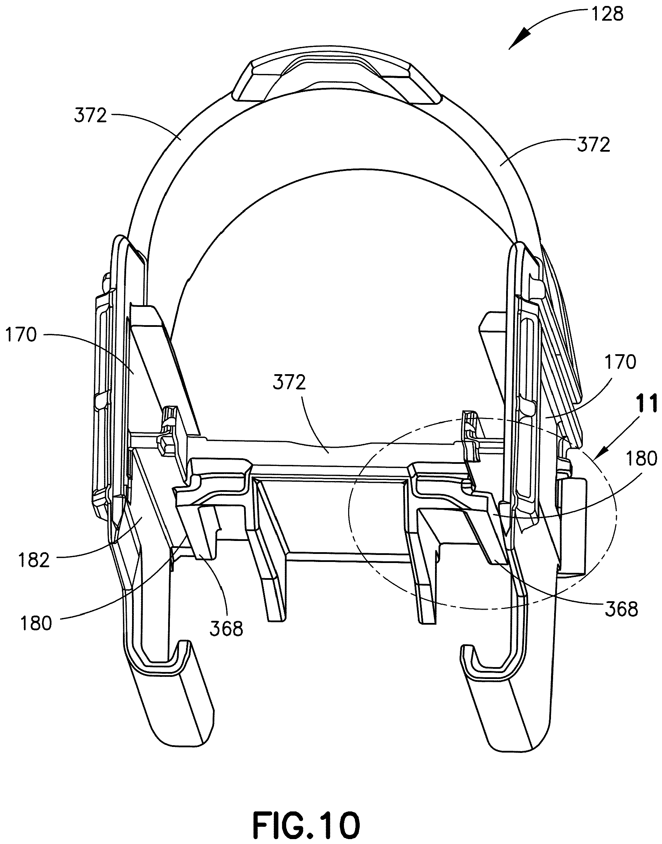

Each of the sidewalls 326 of the motor body 159 includes a contact surface 160 for engaging with the cassette body 128. The contact surface 160 is configured to slide along a corresponding contact surface 180 (see FIGS. 10-11) of the cassette body 128. The contact surfaces 160 of the motor body 159 are provided on inside faces of the sidewalls 326 in the illustrated embodiment, but in other embodiments the contact surfaces 160 may be provided on the outside of the sidewalls 326 with corresponding modifications made to the cassette body 128. The contact surfaces 160 of the motor body 159 may be planar, substantially vertical surfaces, though other profiles are within the scope of the present disclosure. The contact surfaces 160 of the motor body 159 may be configured for continuous contact with the corresponding contact surfaces 180 of the cassette body 128 along the length of the contact surfaces 160, 180. In some embodiments, the contact surfaces 160, 180 may be configured for intermittent contact along the length of the contact surfaces 160, 180. In non-limiting embodiments, contact surface 180 has a slope that provides for ease of assembly of first subassembly 110 and second subassembly 140.

On the opposite side of each sidewall 326 relative to the contact surface 160 (i.e., on the outside face of the sidewall 326 in the illustrated embodiment), the motor body 159 includes at least one protrusion 162 configured to maintain sliding contact between the contact surface 160 of the motor body 159 and the corresponding contact surface 180 of the cassette body 128. In particular, the protrusion 162 may be located and sized so as to engage a corresponding wall surface 182 (see FIG. 9-10) of the cassette body 128, which in turn forces the contact surface 160 of the motor body 159 into contact with the corresponding contact surface 180 of the cassette body 128. Thus, lateral slop between the contact surfaces 160 of the motor body 159 and the contact surfaces 180 of the cassette body 128 is eliminated. Even in the event of deformation of the motor body 159 prior to assembly, or imprecise manufacturing of the motor body 159, the contact surfaces 160 and protrusions 162 interact with the cassette body 128 to hold the motor body 159 in the intended shape to ensure smooth assembly and operation of the device 100. In various embodiments, the at least one protrusion 162 may include a single protrusion or multiple protrusions provided at intervals along the length of the sidewall 326. The at least one protrusion 162 may include a curved outer surface, as shown in the accompanying drawings, but other profiles are within the scope of the present disclosure.

Referring to FIGS. 9-11, the cassette body 128 is generally U-shaped and configured to fit within the lower housing shell 112 and receive the syringe 116 and various other components as described herein in connection with FIGS. 1-4. The cassette body 128 includes opposing sidewalls 170, the inside of which may include the wall surface 182 to which the protrusions 162 of the motor body 159 engage. The cassette body 128 further includes the openings 330 that receive the corresponding cassette clips 328 (see FIG. 6) of the motor body 159 when the device 100 is assembled. The cassette body 128 includes a needle cover clip 364 that engage the needle cover 124 to restrict the axial movement of the needle cover 124 relative to the cassette body 128. The cassette body 128 further includes motor body ribs 368 and upper housing shell ribs 370, which are configured to engage corresponding portions of the motor body 159 and the lower housing shell 112, respectively, to aid in the assembly of the device 100. The motor body ribs 368 may include the contact surfaces 180 configured to engage the corresponding contact surfaces 160 of the motor body 159. The contact surfaces 180 may have a complementary profile to the corresponding contact surfaces 160 of the motor body 159. For example, the contact surfaces 180 may be substantially planar, vertical surfaces as shown in the accompanying drawings. In some embodiments, the contact surfaces 180 extend substantially the entire length of the motor body ribs 368. The cassette body 128 also includes syringe holder stops 372, which are configured to engage portions of the syringe holder 118 to limit the axial movement of the syringe holder 118 relative to the cassette body 128.

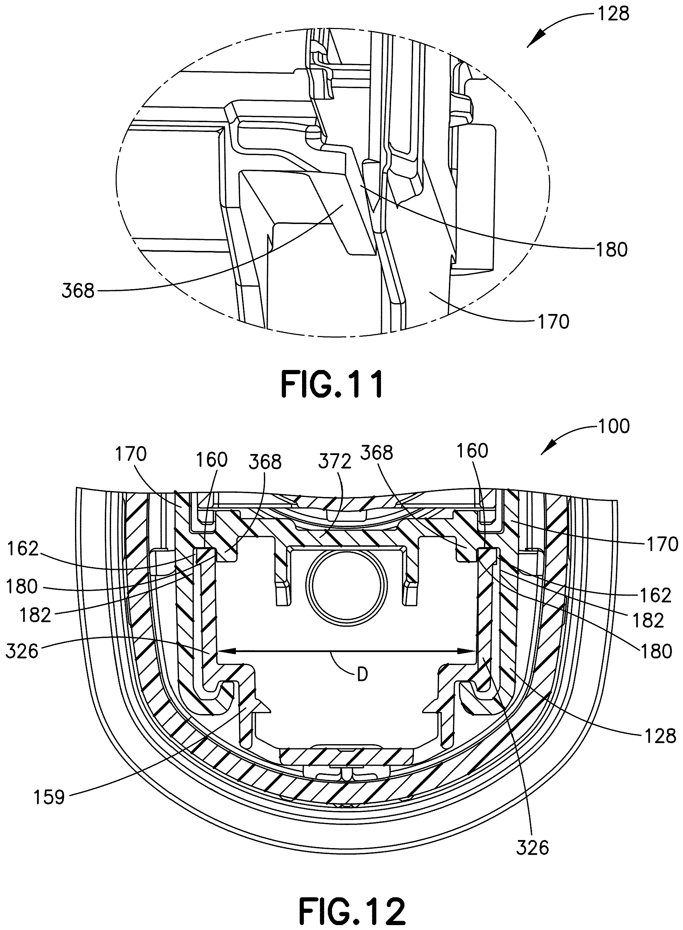

Referring now to FIG. 12, a cross section of the device is illustrated to show the interaction between the cassette body 128 and the motor body 159. Each of the protrusions 162 of the motor body 159 engages the corresponding wall surface 182 of the cassette body 128. The interaction of the protrusions 162 and the wall surfaces 182 forces the contact surfaces 160 of the motor body 159 into engagement with the contact surfaces 180 of the cassette body 128, thereby defining a predetermined distance D between inside faces of the sidewalls 326 of the motor body 159. Likewise, interaction of the protrusions 162 and the wall surfaces 182 forces the sidewalls 326 of the motor body 159 parallel to one another. Due to the cassette body 128 maintaining the shape of the motor body 159 in this manner, assembly of the device 100 is improved, friction between interacting components is reduced, and more reliable drug delivery is achieved. For example, consistency of the predetermined distance D prevents jamming of the lever actuation member 130 against the sidewalls 326 of the motor body 159.

Although the present disclosure has been described in detail for the purpose of illustration based on what is currently considered to be the most practical and preferred embodiments, it is to be understood that such detail is solely for that purpose and that the present disclosure is not limited to the disclosed embodiments, but, on the contrary, is intended to cover modifications and equivalent arrangements that are within the spirit and scope of the appended claims. For example, it is to be understood that the present disclosure contemplates that, to the extent possible, one or more features of any embodiment can be combined with one or more features of any other embodiment.

## Claims

1. A drug delivery device comprising:
an upper housing shell;
a lower housing shell;
a syringe comprising a barrel, a stopper, and a needle, at least a portion of the syringe positioned within the lower housing shell;
a drive assembly comprising a plunger body configured to move the stopper within the barrel upon actuation of the drive assembly;
a cassette body configured to receive the syringe; and
a motor body configured to engage the cassette body,
wherein the motor body comprises:
a pair of sidewalls; and
a contact surface provided on each of the pair of sidewalls, the contact surface engaging a corresponding contact surface of the cassette body.

2. The drug delivery device of claim 1, wherein the contact surface of the motor body comprises a planar, substantially vertical surface.

3. The drug delivery device of claim 1 or 2, wherein the corresponding contact surface of the cassette body comprises a planar, substantially vertical surface.

4. The drug delivery device of any of claims 1-3, wherein the contact surface of the motor body continuously contacts the corresponding contact surface of the cassette body contact along a length of the contact surface of the motor body.

5. The drug delivery device of any of claims 1-4, wherein the cassette body comprises at least one motor body rib comprising the contact surface of the cassette body.

6. The drug delivery device of claim 5, wherein the contact surface of the cassette body extends substantially the entire length of the at least one motor body rib.

7. The drug delivery device of any of claims 1-6, wherein the at least contact surface of the motor body is provided on an inside face of the sidewall of the motor body.

8. The drug delivery device of any of claims 1-7, wherein the motor body comprises at least one protrusion configured to engage a corresponding wall surface of the cassette body.

9. The drug delivery device of claim 8, wherein the cassette body comprises a pair of sidewalls comprising the corresponding wall surface.

10. The drug delivery device of claim 8 or 9, wherein the protrusion comprises a curved outer surface.

11. The drug delivery device of any of claims 8-10, wherein interaction of the at least one protrusion and the corresponding wall surface of the cassette body forces the contact surface of the motor body into engagement with the contact surface of the cassette body.

12. The drug delivery device of any of claims 8-11, wherein contact between the at least one protrusion and the corresponding wall surface of the cassette body defines a predetermined distance between inside faces of the sidewalls of the motor body.

13. The drug delivery device of any of claims 8-12, wherein contact between the at least one protrusion and the corresponding wall surface of the cassette body forces the sidewalls of the motor body parallel to one another.

14. The drug delivery device of any of claims 8-13, wherein the at least one protrusion and the contact surface of the motor body is provided on opposite sides of the sidewall of the motor body.

15. The drug delivery device of any of claims 8-14, wherein the at least one protrusion is provided on an outside face of the sidewall of the motor body.
